# EUROPEAN PATENT APPLICATION

(11) **EP 4 585 292 A1**
(43) Date of publication of application: **16.07.2025**
(21) Application number: 23862879.6
(22) Date of filing: 10.08.2023
(51) Int. Cl.: B01D 53/04, B01D 53/28, B01J 20/22

(54) **ADSORBENT AND METHOD FOR USING SAME**

(30) Priority: 09.09.2022 JP 2022143369
(71) Applicant: Panasonic Intellectual Property Management Co., Ltd., Kadoma-shi, Osaka 571-0057 (JP)
(72) Inventor: OTA Tomohiro, Kadoma-shi, Osaka 571-0057 (JP); TAGUCHI Kiyoshi, Kadoma-shi, Osaka 571-0057 (JP); TAKEDA Kenyu, Kadoma-shi, Osaka 571-0057 (JP)
(74) Representative: Grünecker Patent- und Rechtsanwälte PartG mbB
(86) International application number: PCT/JP2023/029379
(87) International publication number: WO 2024/053339

(57) **Abstract**

An adsorbent according to the present disclosure is an adsorbent for removing an adsorbate from a moisture-containing gas which is a gas containing moisture and the adsorbate, wherein the adsorbent includes a metal-organic framework, the metal-organic framework includes at least one metal ion selected from an aluminum ion, an iron ion, and a copper ion, trimesic acid, and an aromatic carboxylic acid compound, and the aromatic carboxylic acid compound is a benzoic acid compound represented by formula (1) or an isophthalic acid compound represented by formula (2). 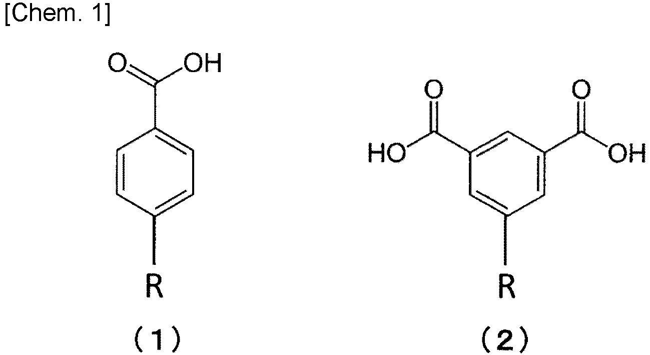 R in formula (1) and formula (2) is one of a methyl group, a fluoro group, a chloro group, an iodo group, and a bromo group.

## Description

### TECHNICAL FIELD

The present disclosure relates to an adsorbent and a use method therefor.

### BACKGROUND ART

Patent Literature 1 discloses a technique for bringing a gas containing dimethyl sulfide into contact with a metal-organic framework composed of a copper ion and trimesic acid, thereby removing dimethyl sulfide from the gas.

### CITATION LIST

### Patent Literature

Patent Literature 1: WO 2017/150019 A1

### Non Patent Literature

Non Patent Literature 1: Journal of Material of Chemistry A, 1, 2013, 6737-6745
Non Patent Literature 2: Journal of American Chemical Society, 136, 2014, 16978-16981
Non Patent Literature 3: Microporous Mesoporous Material, 326, 2021, 11357-11367

### SUMMARY OF INVENTION

### Technical Problem

The present disclosure provides an adsorbent that can reduce the amount of the adsorbent to be packed in an adsorber to downsize the adsorber.

### Solution to Problem

An adsorbent according to the present disclosure is an adsorbent for removing an adsorbate from a moisture-containing gas which is a gas containing moisture and the adsorbate, wherein the adsorbent includes a metal-organic framework, the metal-organic framework includes at least one metal ion selected from an aluminum ion, an iron ion, and a copper ion, trimesic acid, and an aromatic carboxylic acid compound, and the aromatic carboxylic acid compound is a benzoic acid compound represented by formula (1) or an isophthalic acid compound represented by formula (2). 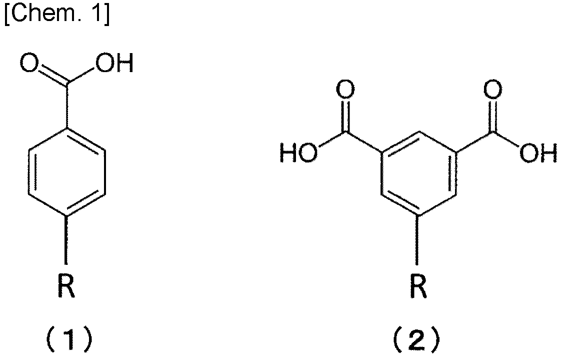 R in formula (1) and formula (2) is one of a methyl group, a fluoro group, a chloro group, an iodo group, and a bromo group.

### Advantageous Effects of Invention

The adsorbent according to the present disclosure includes a metal-organic framework having a hydrophobic functional group having a size that is the same as or smaller than that of a carboxyl group, such as a methyl group, a fluoro group, a chloro group, an iodo group, and a bromo group.

Accordingly, the amount of moisture adsorbed by the metal-organic framework is reduced by inhibiting entry of moisture into the inside of the metal-organic framework without causing a decrease in specific surface area due to pore blockage. Even if the gas containing the adsorbent contains moisture, a decrease in the adsorption capacity of the metal-organic framework due to degradation by moisture can be suppressed.

Therefore, it is possible to provide an adsorbent that can reduce the amount of the adsorbent to be packed in the adsorber to downsize the adsorber.

### BRIEF DESCRIPTION OF THE DRAWINGS

FIG. 1 is a schematic diagram showing the configuration of an adsorber according to Embodiment 1.
FIG. 2 is a characteristic diagram showing an X-ray diffraction pattern of Cu-BTC in Embodiment 1.
FIG. 3 is a characteristic diagram showing a nitrogen adsorption isotherm of Cu-BTC in Embodiment 1.
FIG. 4 is a characteristic diagram showing a water adsorption isotherm of Cu-BTC in Embodiment 1.

### DESCRIPTION OF EMBODIMENTS

### (Findings on which the present disclosure is based, etc.)

At the time when the inventors came to conceive of the present disclosure, as an adsorbent for removing a sulfur compound from a gas containing the sulfur compound, there was Ag zeolite having Ag ions introduced into the ion exchange site of zeolite.

Thus, even sulfur compounds that were difficult to adsorb could be effectively removed. However, since Ag, which is a raw material, is expensive, a cost problem was left unsolved.

Meanwhile, as an adsorbent for removing a sulfur compound from a gas containing the sulfur compound, there was a metal-organic framework composed of a copper ion and trimesic acid disclosed in Patent Literature 1.

This material has a high copper ion content in its structure and a high specific surface area, and thus can effectively remove even sulfur compounds that are difficult to adsorb, and the material is a material whose cost is low compared to Ag zeolite.

However, as disclosed in Non Patent Literature 1, the metal-organic framework having a copper ion and trimesic acid has low humidity resistance and low water resistance, so that there is a problem that a crystal structure is destroyed by humidity, water vapor, and water, and the gas occlusion performance deteriorates when the metal-organic framework is exposed to moisture for a long time.

Therefore, in Non Patent Literature 2, it is proposed to decrease the hygroscopicity and improve the humidity resistance by treating the surface of the metal-organic framework with a binder such as silicone, but when doing so, there is a problem that the adsorption capacity of the metal-organic framework is reduced due to the combination with the binder.

In Non Patent Literature 3, it is proposed to reduce the amount of moisture adsorbed by the metal-organic framework, by introducing a hydrophobic functional group such as naphthalene into the organic moiety of the metal-organic framework, but when doing so, there is a problem that the specific surface area of the metal-organic framework is reduced due to the introduction of the bulky functional group and the adsorption capacity for the target adsorbate is reduced.

Under these circumstances, the inventors have obtained an idea that by introducing a hydrophobic functional group having a small steric hindrance into the structure of a metal-organic framework, the amount of moisture adsorbed by the metal-organic framework can be reduced without causing a decrease in adsorption capacity due to blockage of the pores of the metal-organic framework, thereby suppressing degradation of the metal-organic framework due to moisture.

The inventors have found a problem that, in order to realize this idea and downsize an adsorber, since simply introducing a hydrophobic functional group having a small steric hindrance into a metal-organic framework composed of terephthalic acid and a metal oxide cluster as in Non Patent Literature 3 cannot create a hydrophobic atmosphere in the vicinity of a metal ion site serving as an adsorption active point and sufficient hydrophobicity cannot be obtained, it is necessary to design a metal-organic framework such that a hydrophobic functional group and a metal ion site are close to each other. In order to solve this problem, the inventors have come to constitute the subject matter of the present disclosure.

Therefore, the present disclosure provides an adsorber that can be downsized and a use method therefor.

### (Embodiment 1)

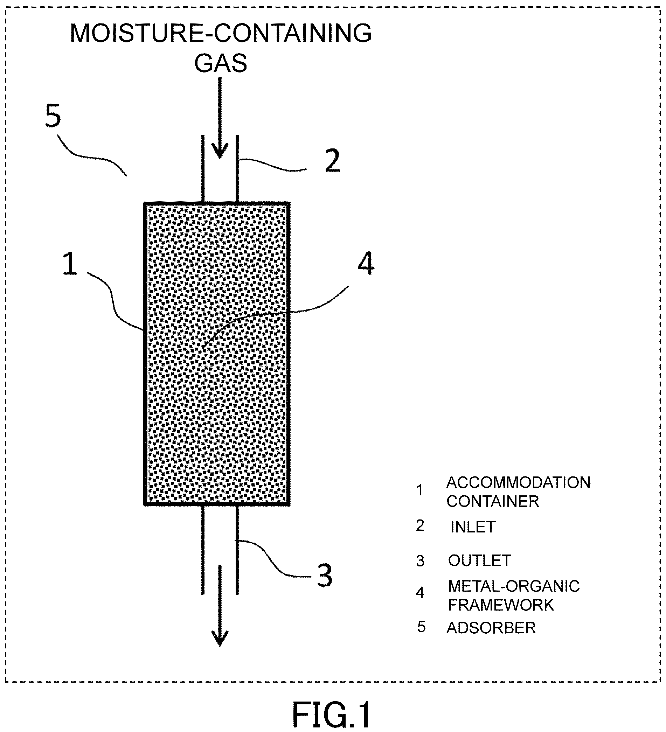

Hereinafter, Embodiment 1 will be described with reference to FIG. 1 to FIG. 4 and (Table 1) to (Table 3).

### [1-1. Configuration]

As shown in FIG. 1, a metal-organic framework 4 used in an adsorbent of the present embodiment is packed (loaded) in an accommodation container 1. The accommodation container 1 is provided with an inlet 2, at one end in the longitudinal direction thereof, for allowing a moisture-containing gas, which is a gas containing moisture and an adsorbate, to flow into the accommodation container 1, and is provided with an outlet 3, at another end thereof, for allowing the moisture-containing gas, from which the adsorbate has been removed by the metal-organic framework 4, to flow out from the inside of the accommodation container 1.

An adsorber 5 is configured such that a moisture-containing gas, which is a gas containing moisture and an adsorbate, flows into the inlet 2 of the accommodation container 1 in which the metal-organic framework 4 is packed (loaded) and the moisture-containing gas from which the adsorbate has been removed by the metal-organic framework 4 packed (loaded) in the accommodation container 1 flows out from the inside of the accommodation container 1 to the outlet 3.

The metal-organic framework 4 according to the present embodiment is a metal-organic framework including at least one metal ion selected from an aluminum ion, an iron ion, and a copper ion, trimesic acid (C₉H₆O₆) having a structure in which carboxylic acids are bonded to the 1, 3, and 5-positions of a benzene ring and also called 1,3,5-benzene tricarboxylic acid, and an aromatic carboxylic acid compound.

The aromatic carboxylic acid compound in the present embodiment is a benzoic acid compound represented by formula (1) or an isophthalic acid compound represented by formula (2). 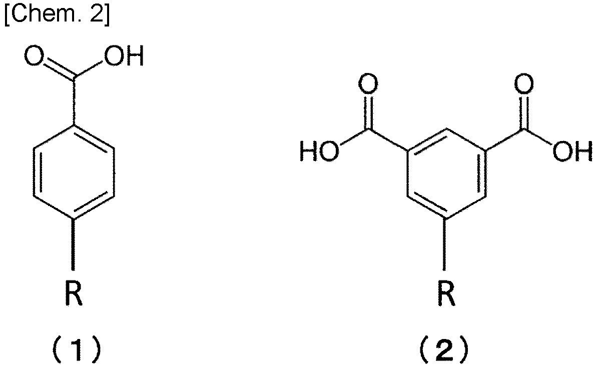 R in Formula (1) and Formula (2) is one of a methyl group, a fluoro group, a chloro group, an iodo group, and a bromo group.

The metal-organic framework 4 is a framework in which, in a metal-organic framework composed of a metal ion and trimesic acid and called MOF-199, a part of trimesic acid is replaced by the benzoic acid compound represented by formula (1) or the isophthalic acid compound represented by formula (2).

The metal-organic framework 4 may be in powder form. The powder is suitable, for example, to have a BET specific surface area of 1000 m²/g or more. The average particle shape of primary particles of the metal-organic framework 4 is not particularly limited, and is 1 µm or more and 30 µm or less, for example.

The metal-organic framework has a uniform skeletal structure in which molecules constituting a three-dimensional skeleton of the metal-organic framework are regularly arranged, and thus has a high specific surface area. By appropriately selecting and combining an organic ligand and a metal ion, physical properties or chemical properties such as pore diameter, pore structure, and surface function can be precisely set. Therefore, compared to conventional adsorbents such as zeolite, the metal-organic framework has a very high flexibility in design.

In order to both maintain the specific surface area of the metal-organic framework and impart hydrophobicity, the present inventors have decided to introduce a hydrophobic functional group having a small steric hindrance compared to carboxyl groups, such as a methyl group, a fluoro group, a chloro group, an iodo group, and a bromo group, into the metal-organic framework.

As a method for introducing the hydrophobic functional group, the present inventors have focused on benzoic acid, in which one carboxylic acid is coordinated to a benzene ring, and an isophthalic acid compound, in which two carboxylic acids are coordinated to a benzene ring, in contrast to trimesic acid in which three carboxylic acids are coordinated to a benzene ring.

In the metal-organic framework 4, a part of trimesic acid included in the MOF-199 structure is replaced by replacement with a benzoic acid compound or isophthalic acid compound containing one of a methyl group, a fluoro group, a chloro group, an iodo group, and a bromo group. Thus, the metal-organic framework 4 has one of a methyl group, a fluoro group, a chloro group, an iodo group, and a bromo group while maintaining the MOF-199 structure.

### [1-2. Operation]

The operation and effects of the adsorber 5 of the present embodiment configured as described above will be described below.

The metal-organic framework 4 containing one of a methyl group, a fluoro group, a chloro group, an iodo group, and a bromo group is accommodated (packed) in the inside of the accommodation container 1 of the adsorber 5.

From a supply source (not shown) of a moisture-containing gas which is a gas containing moisture and an adsorbate, the moisture-containing gas is supplied to the inlet 2. Then, the moisture-containing gas supplied to the inlet 2 passes through the accommodation container 1 while coming into contact with the metal-organic framework 4 inside the accommodation container 1. The adsorbate is removed from the moisture-containing gas by the metal-organic framework 4, so that the moisture-containing gas from which the adsorbate has been removed is discharged from the outlet 3.

An example of the moisture-containing gas in the present embodiment is a hydrocarbon fuel such as city gas, natural gas, or LPG. The hydrocarbon fuel has a sulfur adsorbate added for security purposes such that the hydrocarbon fuel can be quickly detected when the hydrocarbon fuel leaks. An example of the adsorbate contained in the moisture-containing gas is tetrahydrothiophene (THT).

The functional group contained in the metal-organic framework 4, such as a methyl group, a fluoro group, a chloro group, an iodo group, and a bromo group, has a size that is the same as or smaller than that of a carboxyl group. At some of locations where carboxyl groups are present in a conventional metal-organic framework including only a metal ion and trimesic acid, hydrophobic functional groups having a size that is the same as or smaller than that of a carboxyl group are present in the metal-organic framework 4.

Therefore, it can be considered that the metal-organic framework 4 does not cause a decrease in specific surface area due to pore blockage, and that entry of moisture into the inside of the metal-organic framework is more inhibited than in a conventional metal-organic framework including only a metal ion and trimesic acid.

When a metal-organic framework is exposed to moisture for a long period of time, the crystal structure of the metal-organic framework is destroyed, and the adsorption capacity for the adsorbate is reduced due to a decrease in specific surface area, so that it is assumed that the metal-organic framework of the present disclosure can suppress deterioration of the metal-organic framework due to moisture in the moisture-containing gas.

### [1-3. Effects, etc.]

As described above, in the present embodiment, the metal-organic framework 4 is an adsorbent for removing an adsorbate from a moisture-containing gas which is a gas containing moisture and the adsorbate, and has at least one hydrophobic functional group having a size that is the same as or smaller than that of a carboxyl group, such as a methyl group, a fluoro group, a chloro group, an iodo group, and a bromo group.

The metal-organic framework 4 contains at least one metal ion selected from an aluminum ion, an iron ion, and a copper ion, trimesic acid, and an aromatic carboxylic acid compound, and the aromatic carboxylic acid compound is a benzoic acid compound represented by formula (1) or an isophthalic acid compound represented by formula (2). 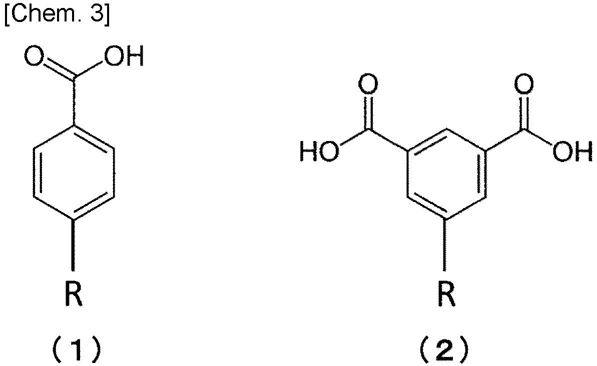 R in formula (1) and formula (2) is one of a methyl group, a fluoro group, a chloro group, an iodo group, and a bromo group.

Accordingly, the amount of moisture adsorbed by the metal-organic framework 4 is reduced by inhibiting entry of moisture into the inside of the metal-organic framework 4 without causing a decrease in specific surface area due to pore blockage. Even if the gas containing the adsorbent contains moisture, a decrease in the adsorption capacity of the metal-organic framework 4 due to degradation by moisture can be suppressed.

Therefore, it is possible to provide an adsorbent (metal-organic framework 4) that can reduce the amount of the adsorbent to be packed in the adsorber 5 to downsize the adsorber 5.

### EXAMPLES

Examples will be described below.

### (Synthesis method)

Many metal-organic frameworks can be synthesized by a known solvothermal method (i.e., hydrothermal synthesis method). For example, a metal ion source and an organic ligand are added to a solvent such as ethylene glycol to prepare a starting material solution.

Next, the starting material solution is heated to grow crystals of a metal-organic framework. Examples of the metal ion source include copper sulfate hydrate and iron sulfate hydrate. A product having the synthesized metal-organic framework is washed with a washing liquid to remove the residual raw materials from the product. An example of the washing liquid is methanol with a low viscosity and a low boiling point. After washing, a powdered metal-organic structure is obtained through solid-liquid separation and drying.

Hereinafter, an example of a synthesis method for the metal-organic framework according to Embodiment 1 will be described in detail. In the present example, Cu-BTC-CH3-BA synthesized using copper sulfate pentahydrate as a metal source and 4-methylbenzoic acid as a benzoic acid compound, will be described.

For Cu-BTC-CH3-BA, anhydrous copper sulfate (3.6 mmol), trimesic acid (1.6 mmol), and 4-methylbenzoic acid (0.48 mmol) were mixed in ethylene glycol (40 mL) to prepare a raw material solution.

In the raw material solution, the molar ratio of 4-methylbenzoic acid to trimesic acid was 30%. The raw material solution was poured into a sealed container and then heated at 120°C for 24 hours to obtain a product. Furthermore, the obtained product was filtered by suction to obtain a precipitate. The obtained precipitate was put into a methanol solution and washed by stirring for 24 hours, and the precipitate was again obtained by suction filtration. Finally, the washed precipitate was dried at 120°C.

Thus, Cu-BTC-CH3-BA (Example 1) was obtained. In the same synthesis method as for Cu-BTC-CH3-BA, Cu-BTC-CH3-ISO (Example 2) was obtained using 5-methylisophthalic acid (0.48 mmol) instead of 4-methylbenzoic acid, and Cu-BTCCI-BA (Example 3) was obtained using 4-chlorobenzoic acid (0.48 mmol) instead of 4-methylbenzoic acid.

Furthermore, as samples for comparison, Cu-BTC-BA (Comparative Example 1) was obtained by mixing anhydrous copper sulfate (3.6 mmol), trimesic acid (2 mmol), and benzoic acid (0.48 mmol) in ethylene glycol (40 mL) and then performing the same procedure, and Cu-BTC-ISO (Comparative Example 2) was obtained by mixing anhydrous copper sulfate (3.6 mmol), trimesic acid (1.6 mmol), and isophthalic acid (0.4 mmol) in ethylene glycol (40 mL) and then performing the same procedure.

### (Structural evaluation)

In order to confirm whether the obtained sample had the same three-dimensional structure as Cu-BTC, Cu-BTC-CH3-BA of Example 1 was subjected to powder X-ray diffraction analysis.

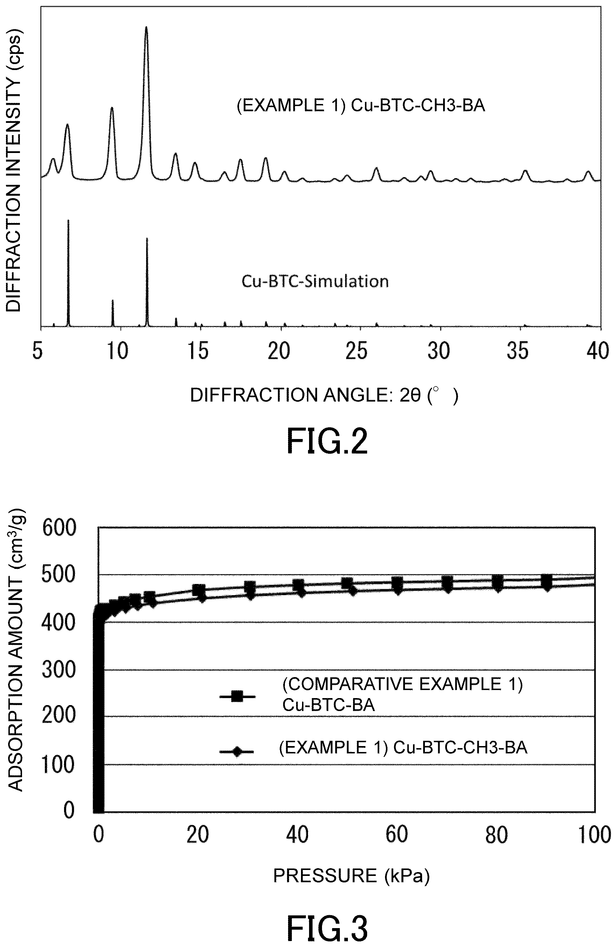

FIG. 2 is a characteristic diagram showing a powder X-ray diffraction pattern of the sample. In FIG. 2, the vertical axis indicates a diffraction intensity and the horizontal axis indicates a diffraction angle 2θ.

Powder X-ray diffraction analysis pattern simulation of Cu-BTC is based on the Cambridge Structural Database, deposit number 112954. As is obvious from FIG. 2, only Cu-BTC-derived peaks were identified in Cu-BTC-CH3-BA.

From this, it was found that Cu-BTC-CH3-BA has the same three-dimensional crystal structure as Cu-BTC and contains no other impurity crystals.

Next, the nitrogen adsorption isotherms of Cu-BTC-CH3-BA of Example 1 and Cu-BTC-BA of Comparative Example 1 at 77 k were measured. Before the measurement, the samples were heated under vacuum for 12 hours at a temperature condition of 150°C to remove the residual solvent and adsorbed moisture.

FIG. 3 is a characteristic diagram showing the nitrogen adsorption isotherms. In FIG. 3, the vertical axis indicates the amount of nitrogen adsorbed and the horizontal axis indicates an adsorption equilibrium pressure. As shown in FIG. 3, the isotherm of each sample was confirmed to be an IUPAC type I isotherm attributable to the presence of micropores.

(Table 1) shows BET specific surface areas calculated using the BET formula from the adsorption isotherms in FIG. 3.

**[Table 1]**

| Sample | BET specific surface area (m²/g) |
|---|---|
| (Comparative Example 1) Cu-BTC-BA | 1,843 |
| (Example 1) Cu-BTC-CH3-BA | 1,820 |

As shown in (Table 1), the specific surface area of Cu-BTC-CH3 of Example 1 was a value similar to that of the specific surface area of Cu-BTC-BA of Comparative Example 1 in which only benzoic acid having no hydrophobic functional group was introduced.

From this, it can be considered that when a benzoic acid compound is introduced, blockage of the pores of the metal-organic framework due to the introduction of a methyl group does not occur.

(Table 2) shows the BET specific surface areas of Cu-BTC-ISO of Comparative Example 2, Cu-BTC-CH3-ISO of Example 2, and Cu-BTC-Cl-BA of Example 3.

**[Table 2]**

| Sample | BET specific surface area (m²/g) |
|---|---|
| (Comparative Example 2) Cu-BTC-ISO | 1,722 |
| (Example 2) Cu-BTC-CH3-ISO | 1,729 |
| (Example 3) Cu-BTC-Cl-BA | 1,714 |

As shown in (Table 2), the specific surface areas of Cu-BTC-ISO of Comparative Example 2 and Cu-BTC-CH3-ISO of Example 2 are similar values.

From this, it can be considered that even when an isophthalic acid compound is introduced, blockage of the pores of the metal-organic framework due to the introduction of a methyl group does not occur.

The specific surface area of Cu-BTC-Cl-BA of Example 3 is a value slightly lower than that of Cu-BTC-BA of Comparative Example 1.

From this, it can be considered that even when halogen is introduced as a hydrophobic functional group into the metal-organic framework, almost no decrease in specific surface area due to pore blockage occurs.

Subsequently, the water adsorption isotherms of Cu-BTC-CH3-BA of Example 1 and Cu-BTC-BA of Comparative Example 1 at 298 K were measured. Before the measurement, the samples were heated under vacuum for 12 hours at a temperature condition of 150°C to remove the residual solvent and adsorbed moisture.

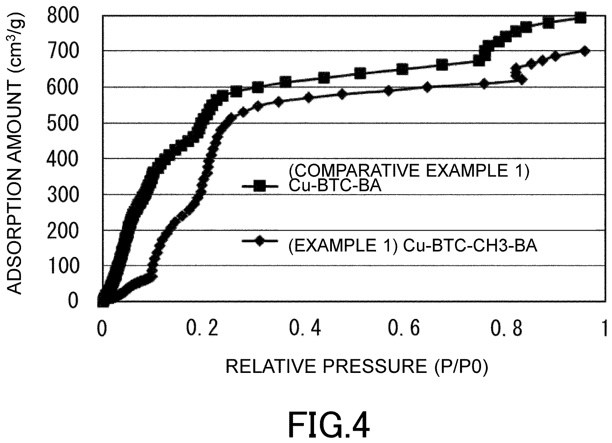

FIG. 4 is a characteristic diagram showing the water adsorption isotherms. In FIG. 4, the vertical axis indicates the amount of moisture adsorbed and the horizontal axis indicates the relative pressure at which adsorption equilibrium was reached. Since the saturated water vapor pressure at 298 K is 3.2 kPa, the relative pressure is (P/3.2 kPa).

As shown in FIG. 4, the moisture adsorption capacity of Cu-BTC-CH3-BA of Example 1 is lower than that of Cu-BTC-BA of Comparative Example 1, and significantly lower especially in the range where the relative pressure is 0 to 0.25.

From this, when even a low-bulk hydrophobic functional group such as a methyl group is introduced to the MOF-199 structure, an effect of reducing the moisture adsorption capacity of the metal-organic framework is obtained.

In this example, the methyl group was introduced by 30% with respect to trimesic acid, and it can be considered that increasing the amount of methyl group introduced widens the range of relative pressure where the moisture adsorption capacity can be significantly reduced.

From the above, in the metal-organic framework of the present disclosure in which hydrophobicity is imparted to the surface of the metal-organic framework by mixing an organic ligand having a hydrophobic functional group whose steric hindrance is similar to that of a carboxyl group, such as a methyl group, a fluoro group, a chloro group, an iodo group, and a bromo group, during synthesis, destruction of the metal-organic framework skeleton due to the treatment for introducing the functional group and a significant decrease in specific surface area due to pore blockage by the introduced functional group, did not occur.

In addition, it was possible to reduce the moisture adsorption capacity by introducing even a low-bulk hydrophobic functional group such as a methyl group into the MOF-199 structure.

### (Sulfur adsorption performance evaluation)

(Table 3) shows the THT adsorption capacity of each sample at 15 ppm.

**[Table 3]**

| Sample | THT adsorption capacity at 15 ppm (wt%) |
|---|---|
| (Comparative Example 1) Cu-BTC-BA | 11.8 |
| (Comparative Example 2) Cu-BTC-ISO | 9.9 |
| (Example 1) Cu-BTC-CH3-BA | 13.9 |
| (Example 2) Cu-BTC-CH3-ISO | 9.8 |
| (Example 3) Cu-BTC-Cl-BA | 10.1 |

The THT adsorption capacity was evaluated by introducing the sample and THT gas into a bag, leaving the bag for 24 hours, analyzing the THT concentration in the bag to measure the actual THT adsorption capacity for multiple equilibrium concentrations, calculating an adsorption isotherm using the Langmuir equation assuming that adsorbate molecules were adsorbed to a monomolecular layer, and calculating the adsorption capacity at 15 ppm.

As is obvious from (Table 3), Cu-BTC-CH3-BA of Example 1 in which the methyl group was introduced exhibited a higher THT adsorption capacity than Cu-BTC-BA of Comparative Example 1 in which no methyl group was introduced.

From this, the moisture adsorption capacity of Cu-BTC-CH3-BA is reduced compared to that of Cu-BTC-BA, but the adsorption amount of the target sulfur component is not reduced.

Since Cu-BTC-CH3-BA is a material that can suppress degradation of a metal-organic framework due to moisture compared to conventional adsorbents, the amount of the adsorbent added on the assumption of degradation can be reduced, and as a result, the amount of the adsorbent required can be reduced.

The reason why Cu-BTC-CH3-BA exhibited a higher adsorption capacity than Cu-BTC-BA is, for example, that moisture of about 2000 to 5000 ppm, which can be considered to be contaminated in the test environment, inhibited the sulfur adsorption of Cu-BTC-BA, while this effect was smaller for Cu-BTC-CH3-BA.

In Cu-BTC-CH3-ISO of Example 2 as well, the adsorption amount of the sulfur component is almost not reduced compared to Cu-BTC-ISO of Comparative Example 2 in which no methyl group was introduced.

In Cu-BTC-Cl-BA of Example 3 as well, the adsorption amount of the sulfur component is slightly reduced due to a decrease in specific surface area, compared to Cu-BTC-BA of Comparative Example 1 in which no chloro group was introduced. From this, it is assumed that even when a methyl group is introduced to the isophthalic acid compound or even when a halogen group is introduced into the benzoic acid compound, it is possible to reduce the amount of the adsorbent as in the case of Cu-BTC-CH3-BA.

As in the present embodiment, the metal-organic framework 4 may have a copper ion, and the adsorbate to be removed may be a sulfur compound.

Due to this, since copper ions are ions that easily adsorb a sulfur compound which is an adsorbate, a higher adsorption capacity is obtained. As a result, it is possible to provide an adsorbent that can further reduce the volume of the adsorbent used in an adsorber to achieve further downsizing.

In addition, as in the present embodiment, the adsorbate to be removed may be THT. Accordingly, the metal-organic framework of the present disclosure has a larger pore diameter than conventional metal-organic frameworks, and thus a higher adsorption capacity is obtained when an adsorbate having a larger molecular size than for conventional metal-organic frameworks is used. As a result, it is possible to provide an adsorbent that can further reduce the volume of the adsorbent used in an adsorber to achieve further downsizing.

In addition, as in the present embodiment, the adsorbent may be used in an environment having a relative humidity of 20% or lower. Accordingly, the adsorbent can be used in a range where the moisture adsorption capacity is significantly reduced compared to conventional metal-organic frameworks, so that a decrease in the adsorption capacity of the metal-organic framework due to degradation by moisture can be more efficiently suppressed. As a result, it is possible to provide an adsorbent that can further reduce the volume of the adsorbent used in an adsorber to achieve further downsizing.

### (Other embodiments)

As described above, Embodiment 1 has been described as an illustration of the technique disclosed in the present application. However, the technique according to the present disclosure is not limited to these, and can also be applied to embodiments obtained by making modifications, replacements, additions, omissions, and the like. Furthermore, the components described in Embodiment 1 above can also be combined to obtain a new embodiment.

Thus, other embodiments will be described below.

In Embodiment 1, 4-methylbenzoic acid and 4-chlorobenzoic acid have been described as examples of the benzoic acid compound containing a hydrophobic functional group. The benzoic acid compound having a functional group may be any compound having a hydrophobic functional group at the meta-position.

Therefore, the benzoic acid compound is not limited to 4-methylbenzoic acid and 4-chlorobenzoic acid. However, when benzoic acid having a hydrophobic functional group with a larger molecular size than a chloro group, such as 4-bromobenzoic acid and 4-iodobenzoic acid, is used as a material to be added during synthesis of the metal-organic framework, the pore size can be reduced, so that the adsorption capacity for inhibitors larger than water can be reduced.

In Embodiment 1, tetrahydrothiophene has been described as an example of the adsorbate. The adsorbate may be any fluid that contains gas.

Therefore, the adsorbate is not limited to tetrahydrothiophene. It should be noted that even when carbon dioxide is used as the adsorbent, a high adsorption capacity is obtained.

### INDUSTRIAL APPLICABILITY

The adsorbent of the present disclosure is an adsorbent for removing an adsorbate from a moisture-containing gas which is a gas containing moisture and the adsorbate, is suitable for applications for reducing the amount of an adsorbent to be packed in an adsorber to downsize the adsorber, and is applicable to an adsorbent for removing a sulfur compound from a fuel gas containing the sulfur compound. Specifically, the present disclosure is applicable to a fuel cell that can convert city gas into hydrogen and generate electricity, etc.

## Claims

1. An adsorbent for removing an adsorbate from a moisture-containing gas which is a gas containing moisture and the adsorbate, wherein
the adsorbent comprises a metal-organic framework,
the metal-organic framework comprises at least one metal ion selected from an aluminum ion, an iron ion, and a copper ion, trimesic acid, and an aromatic carboxylic acid compound, and
the aromatic carboxylic acid compound is a benzoic acid compound represented by formula (1) or an isophthalic acid compound represented by formula (2), 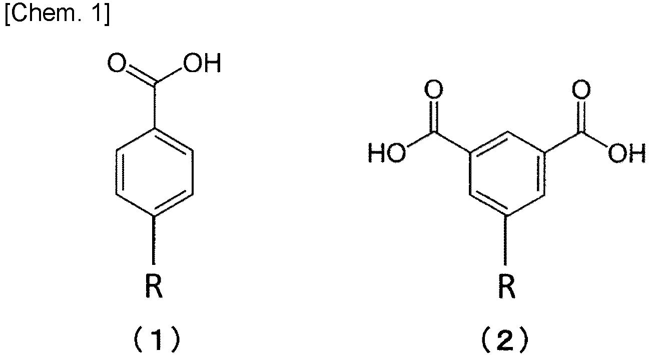
R in formula (1) and formula (2) is one of a methyl group, a fluoro group, a chloro group, an iodo group, and a bromo group.

2. The adsorbent according to claim 1, wherein the adsorbate is a sulfur compound, and the metal ion is a copper ion.

3. An adsorbent use method for removing tetrahydrothiophene from a fluid containing the tetrahydrothiophene, using the adsorbent according to claim 1 or 2.

4. An adsorbent use method using the adsorbent according to claim 1 or 2 in an environment having a relative humidity of 20% or lower.
